# EUROPEAN PATENT APPLICATION

(11) **EP 3 666 191 A1**
(43) Date of publication of application: **17.06.2020**
(21) Application number: 17921138.8
(22) Date of filing: 19.10.2017
(51) Int. Cl.: A61B 6/10, A61B 6/06, G21F 1/04

(54) **RADIATION SHIELD FILTER AND RADIOGRAPHIC APPARATUS FILTER ASSEMBLY COMPRISING SAME**

(30) Priority: 10.08.2017 KR 20170101400
(71) Applicant: Kim, Ki Kyung, Gyeongju-si, Gyeongsangbuk-do 38204 (KR)
(72) Inventor: Kim, Ki Kyung, Gyeongju-si, Gyeongsangbuk-do 38204 (KR)
(74) Representative: Zimmermann, Tankred Klaus
(86) International application number: PCT/KR2017/011609
(87) International publication number: WO 2019/031648

(57) **Abstract**

Disclosed is a filter assembly for a radiographic apparatus, which is positioned at an illumination part of a radiographic apparatus. The disclosed filter assembly for a radiographic apparatus includes a radiation shield filter including silicon dioxide (SiO₂), aluminum oxide (Al₂O₃), boron trioxide (B₂O₃) and sodium oxide (Na₂O), and a radiation alignment filter composed of the same components as the radiation shield filter, laid over the front of the radiation shield filter and having fine holes formed in the surface thereof, and effectively blocks the radiation generated from the radiographic apparatus without greatly affecting the quality of a radiographic image.

## Description

### Technical Field

The present disclosure relates to a radiation shield filter positioned at an illumination part of a radiographic apparatus.

### Background Art

In general, during a surgical (orthopedics, neurosurgery, etc.) operation, a mobile radiographic apparatus (hereinafter referred to as an "X-Ray photographing apparatus"), called a C-arm, is used in the operating room. When a C-arm is provided in the operating room, the affected area may be photographed during the operation to thus confirm in real time whether repositioning or the like is performed normally.

The C-arm emits a smaller amount of radiation during photography than a general fixed X-Ray photographing apparatus, but because of the continuous use thereof during an operation, doctors, nurses and patients located near the C-arm are exposed to a large amount of radiation during the operation.

Accordingly, in order to reduce the amount of radiation emitted from the C-arm, techniques for a radiation shield filter, which may be mounted to the illumination part of the C-arm, are continuously being studied.

With regard thereto, Korean Patent No. 10-1638364 (hereinafter referred to as a "conventional technique") discloses an example of the aforementioned radiation shield filter.

However, since the conventional technique is a liquid filter formed by filling the inside of a case with water and a silver powder, it is difficult to manufacture, and moreover, there is a high risk of breakage thereof.

Meanwhile, although radiation shield filters are manufactured using metal such as lead (Pb) or silver (Ag), having a high radiation-blocking rate, when a radiation shield filter made of lead or silver is applied to the X-ray photographing apparatus, the quality (sharpness, contrast, etc.) of the photographed radiographic image may be excessively degraded, which is undesirable.

### * Citation List

(Patent Document 1) KR10-1638364 B1 (Publication date: July 11, 2016)
(Patent Document 2) KR10-1225241 B1 (Publication date: January 22, 2013)

### Disclosure

### Technical Problem

The present disclosure is intended to provide a radiation shield filter in the form of glass having a high radiation-blocking rate and high durability.

In addition, the present disclosure is intended to provide a filter assembly for a radiographic apparatus, which effectively blocks the radiation generated from the radiographic apparatus without greatly affecting the quality of a radiographic image.

### Technical Solution

There is provided a radiation shield filter positioned at an illumination part of a radiographic apparatus and including silicon dioxide (SiO₂), aluminum oxide (Al₂O₃), boron trioxide (B₂O₃) and sodium oxide (Na₂O).

The radiation shield filter may include 75 to 86 wt% of silicon dioxide, 2 to 4 wt% of aluminum oxide, 9 to 16 wt% of boron trioxide and 3 to 5 wt% of sodium oxide, and the radiation shield filter may further include at least one additive selected from the group consisting of calcium oxide (CaO), chromium oxide (Cr₂O₃), iron oxide (Fe₂O₃), potassium oxide (K₂O), magnesium oxide (MgO) and zirconium oxide (ZrO₂).

In addition, there is provided a filter assembly for a radiographic apparatus including the above radiation shield filter; and a radiation alignment filter composed of the same components as the radiation shield filter, laid over the front of the radiation shield filter and having fine holes formed in the surface thereof.

### Advantageous Effects

According to the present disclosure, the radiation shield filter is provided in the form of glass and has a high radiation-blocking rate and high durability.

In addition, according to the present disclosure, when the filter assembly for a radiographic apparatus is mounted to a radiographic apparatus, it can effectively block the radiation generated from the radiographic apparatus without greatly affecting the quality of a radiographic image.

### Brief Description of Drawings

FIG. 1 shows images captured after application of each of a radiation shield filter according to the present disclosure, a lead filter and a ceramic filter to a radiographic apparatus;
FIG. 2 schematically shows the configuration of a filter assembly 100 for a radiographic apparatus and the radiation XB transmitted through the filter assembly 100 for a radiographic apparatus; and
FIG. 3 shows the radiographic images depending on the thicknesses combination of a radiation shield filter 110 and a radiation alignment filter 130.

### * Description of the Reference Numerals

- 100:: filter assembly for radiographic apparatus
- 110:: radiation shield filter
- 130:: radiation alignment filter
- XB:: radiation

### Mode for Disclosure

Hereinafter, a detailed description will be given of a radiation shield filter and a filter assembly 100 for a radiographic apparatus, made with reference to the appended drawings.

### Radiation shield filter

A radiation shield filter according to the present disclosure (hereinafter referred to as a "radiation shield filter") includes silicon dioxide (SiO₂), aluminum oxide (Al₂O₃), boron trioxide (B₂O₃) and sodium oxide (Na₂O), and is effective at blocking (ionizing) the transmitted radiation.

Specifically, the radiation shield filter may be configured to include 75 to 86 wt% of silicon dioxide, 2 to 4 wt% of aluminum oxide, 9 to 16 wt% of boron trioxide and 3 to 5 wt% of sodium oxide.

Moreover, the radiation shield filter may further include at least one additive selected from the group consisting of calcium oxide (CaO), chromium oxide (Cr₂O₃), iron oxide (Fe₂O₃), potassium oxide (K₂O), magnesium oxide (MgO) and zirconium oxide (ZrO₂).

Silicon dioxide allows the radiation shield filter to be in the form of glass and accounts for the greatest weight percent among the components of the radiation shield filter.

If the weight percent of silicon dioxide exceeds 86 wt%, the weight percents of the radiation-blocking materials (aluminum oxide, boron trioxide) may decrease, and thus the radiation-blocking rate of the radiation shield filter may be lowered. On the other hand, if the weight percent of silicon dioxide is less than 75 wt%, the weight percents of radiation-blocking materials (aluminum oxide, boron trioxide) may increase, and thus the sharpness of the photographed radiographic image may be lowered.

Aluminum oxide functions to block radiation in the low energy region and to induce curing of the mixture.

If the weight percent of aluminum oxide exceeds 4 wt%, the contrast of the photographed radiographic image increases. Although a certain level of contrast increase aids in identification of the photographed radiographic image, an excessive increase in contrast makes it difficult to identify the photographed radiographic image. On the other hand, if the weight percent of aluminum oxide is less than 2 wt%, the ability to block radiation in the low energy region is lowered.

Boron trioxide functions to block radiation in the energy region higher than the radiation blocked by aluminum oxide.

According to tests conducted by the present inventors, the radiation-blocking rate was increased by 3.5 to 4% with an increase in boron trioxide by 1 wt%. On the other hand, the radiation-blocking rate was decreased by 3.5 to 4% with a decrease in boron trioxide by 1 wt%. As the weight percent of boron trioxide was higher, the photographed radiographic image was darkened.

Sodium oxide functions to increase the thermal shock resistance of the radiation shield filter. Also, when silicon dioxide, aluminum oxide and boron trioxide are solidified, sodium oxide enables these components to be uniformly mixed (distributed).

According to tests conducted by the present inventors, when using 3 to 5 wt% of sodium oxide, desired effects thereof were obtained.

Additives such as calcium oxide (CaO), chromium oxide (Cr₂O₃), iron oxide (Fe₂O₃), potassium oxide (K₂O), magnesium oxide (MgO) and zirconium oxide (ZrO₂) do not affect the radiation-blocking rate of the radiation shield filter, but have an influence on the strength and durability of the radiation shield filter.

### Radiation-blocking test

A) Test method and traceability: The X-ray-blocking rate (1-Cᵢ/C₀) is determined by the ratio of the dose rate values measured when a sample was present (Cᵢ) and when a sample was absent (C₀) between an X-ray irradiator and a reference ionization chamber.
B) Specification of standard equipment used for irradiation

**[Table 1]**

| Device name | Manufacturer and Form | Device number | Expected date of next calibration | Calibration institution |
|---|---|---|---|---|
| X-ray irradiator | YXLON, MG325 | 9040059413 | 2017.4.5. | KAERI |
| Reference ionization chamber | NE2530 | 613 | 2017.6.21. | KRISS |
| Thermometer | WIKA/CTR2000-024 | 024617 | 2017.1.25. | Mokwon University Industry-Academic Cooperation Foundation |
| Barometer | Mensor DPGII-14500 | 290266 | 2017.7.27. | Mokwon University Industry-Academic Cooperation Foundation |

C) Test environment: Temperature (23±5)°C/relative humidity (48±2)% R.H.
D) Test conditions:

**[Table 2]**

| Beam Code | Applied voltage (kV) | Additional filter (mm) | Average energy (keV) |
|---|---|---|---|
| ISO NS60 | 60 | 0.6 Cu | 48 |
| ISO NS80 | 80 | 2.0 Cu | 65 |
| ISO NS100 | 100 | 5.0 Cu | 83 |
| ISO NS120 | 120 | 5.0 Cu + 1.0 Sn | 100 |
| ISO NS150 | 150 | 2.5 Su | 118 |

Inherent filtration : 3.0 mm Be + 3.7 mm AI
- The sample surface and the photon beamline were installed perpendicular to each other.
- Distance between a light source and a measurement instrument: 200 cm
- Distance between a sample surface and a measurement instrument: 5 cm
- Test beam size: about 26 cm in diameter

### Test Example 1

A radiation-blocking test was performed using a radiation shield filter having a thickness of 8.2 mm ± 0.1 mm and composed of 80.2% of silicon dioxide, 2.3% of aluminum oxide, 12.5% of boron trioxide, 3.31% of sodium oxide and 1.69% of other additives. The test results thereof are shown in Table 3 below.

**[Table 3]**

| Beam code | Dose rate C₀ (mGy/h) | Dose rate Cᵢ (mGy/h) | Blocking rate (1-Cᵢ/C₀) | Relative measurement uncertainty (%) |
|---|---|---|---|---|
| ISO NS60 | 30.89 | 16.79 | 0.46 | 5.3 |
| ISO NS80 | 18.31 | 13.00 | 0.29 | 5.4 |
| ISO NS100 | 7.91 | 6.10 | 0.23 | 5.3 |
| ISO NS120 | 9.37 | 7.47 | 0.20 | 5.4 |
| ISO NS150 | 63.33 | 51.37 | 0.19 | 5.3 |

| | | | | |
|---|---|---|---|---|
| * Relative measurement uncertainty: value at approximately 95% confidence level, *k* = 2. | | | | |

### Test Example 2

A radiation-blocking test was performed using a radiation shield filter having a thickness of 9.9 mm ± 0.1 mm and composed of 80.2% of silicon dioxide, 2.3% of aluminum oxide, 12.5% of boron trioxide, 3.31% of sodium oxide and 1.69% of other additives. The test results thereof are shown in Table 4 below.

**[Table 4]**

| Beam code | Dose rate C₀ (mGy/h) | Dose rate Cᵢ (mGy/h) | Blocking rate (1-Cᵢ/C₀) | Relative measurement uncertainty (%) |
|---|---|---|---|---|
| ISO NS60 | 30.89 | 15.23 | 0.51 | 5.3 |
| ISO NS80 | 18.31 | 12.27 | 0.33 | 5.4 |
| ISO NS100 | 7.91 | 5.84 | 0.26 | 5.3 |
| ISO NS120 | 9.37 | 7.91 | 0.23 | 5.3 |
| ISO NS150 | 63.33 | 49.73 | 0.21 | 5.3 |

| | | | | |
|---|---|---|---|---|
| * Relative measurement uncertainty: value at approximately 95% confidence level, *k* = 2. | | | | |

### Radiographic imaging

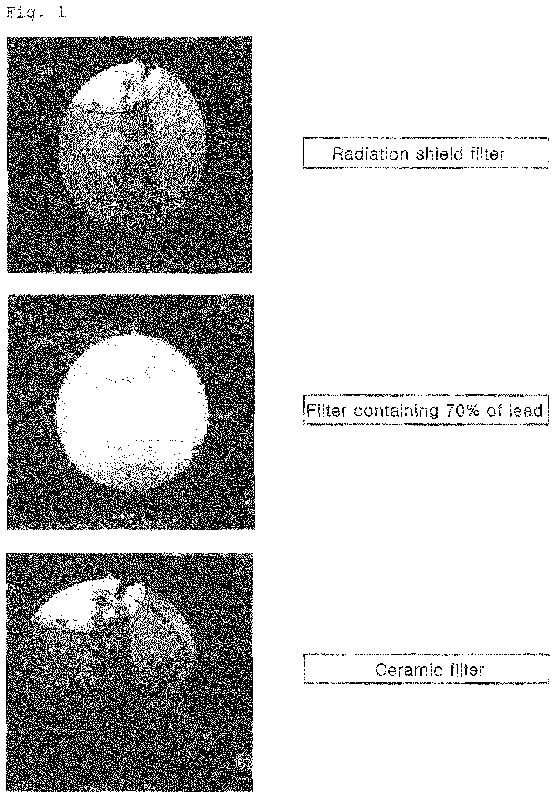

FIG. 1 shows images captured after application of each of the radiation shield filter [having a thickness of 9.9 mm ± 0.1 mm and composed of 80.2% of silicon dioxide, 2.3% of aluminum oxide, 12.5% of boron trioxide, 3.31% of sodium oxide and 1.69% of other additives], a filter containing 70% of lead and a ceramic filter to a radiographic apparatus.

With reference thereto, it can be seen that the radiographic image was very clear even with the naked eye when using the radiation shield filter compared to when using the lead filter and the ceramic filter.

### Filter assembly 100 for radiographic apparatus

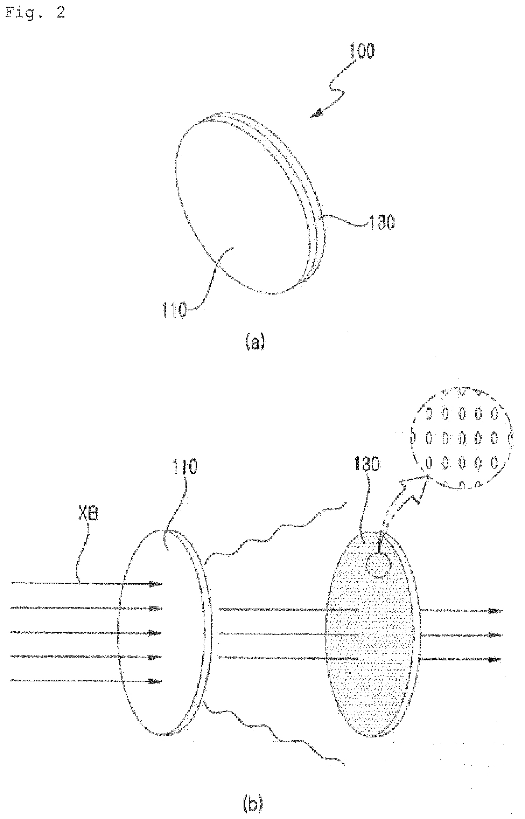

FIG. 2 schematically shows the configuration of the filter assembly 100 for a radiographic apparatus and the radiation XB transmitted through the filter assembly 100 for a radiographic apparatus.

With reference to FIG. 2, the filter assembly 100 for a radiographic apparatus includes a radiation shield filter 100 and a radiation alignment filter 130.

The radiation shield filter 110 functions to ionize (block) most of the dose except the dose necessary to form a radiographic image.

The radiation alignment filter 130 includes the same components as the radiation shield filter 100 and has fine holes formed in the surface thereof. The radiation alignment filter 130 is laid over the front of the radiation shield filter 100 (based on the direction of travel of the radiation XB).

The radiation alignment filter 130 functions to secondarily block the radiation XB. Moreover, the linearity of the radiation XB is corrected, thus improving the radiographic image.

According to tests conducted by the present inventors, when the fine holes formed in the surface of the radiation alignment filter 130 had a size of 40 to 50 *µ*m and an interval of 120 to 170 *µ*m therebetween, an effect of improving the radiographic image was obtained.

Meanwhile, under irradiation conditions of 70 KV and 2.5 mAs for 2.0 sec using a general C-arm, it is preferable for the total thickness of the radiation shield filter 110 and the radiation alignment filter 130 to be 10 mm or less. According to tests conducted by the present inventors, when the total thickness of the radiation shield filter 110 and the radiation alignment filter 130 exceeded 10 mm, the radiographic image was deteriorated.

Table 5 below schematically shows the extent of blocking the radiation and the state of the image depending on changes in the thicknesses of the radiation shield filter 110 and the radiation alignment filter 130 under irradiation conditions of 70 KV and 2.5 mAs for 2.0 sec.

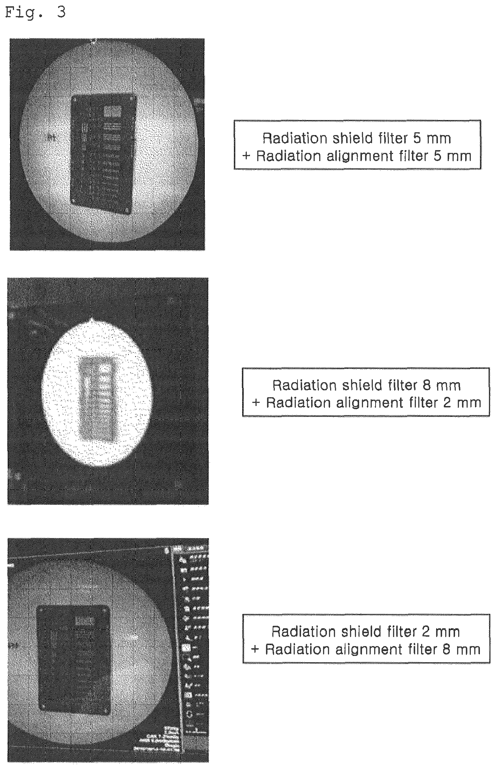

FIG. 3 shows the radiographic images depending on the thickness combination of the radiation shield filter 110 and the radiation alignment filter 130.

**[Table 5]**

| Radiation shield filter | Radiation alignment filter | Remark |
|---|---|---|
| 2 mm | 8 mm | Very low blocking rate / very good image |
| 3 mm | 7 mm | Low blocking rate / good image |
| 4 mm | 6 mm | Appropriate blocking rate / fair image |
| 5 mm | 5 mm | Optimal blocking rate / good image |
| 6 mm | 4 mm | Appropriate blocking rate / fair image |
| 7 mm | 3 mm | High blocking rate / bad image |
| 8 mm | 2 mm | Very high blocking rate / very bad image |

Specifically, when the filter assembly 100 for a radiographic apparatus, in which the radiation shield filter 110 having an appropriate thickness (4 mm to 6 mm) and the radiation alignment filter 130 having an appropriate thickness (4 mm to 6 mm) are combined together, is mounted to a radiographic apparatus, the radiation generated from the radiographic apparatus is effectively blocked but the quality of a radiographic image is not greatly affected.

It is apparent to those skilled in the art that the radiation shield filter and the filter assembly 100 for a radiographic apparatus described above may be variously modified (applied) without departing from the spirit of the invention.

Therefore, the claims of the present disclosure will be broadly interpreted based on the spirit incorporated in the description of the invention and the overall drawings.

### Industrial Applicability

The present disclosure can be applied to radiographic imaging fields.

## Claims

1. A radiation shield filter, positioned at an illumination part of a radiographic apparatus and comprising silicon dioxide (SiO₂), aluminum oxide (Al₂O₃), boron trioxide (B₂O₃) and sodium oxide (Na₂O).

2. The radiation shield filter of claim 1, comprising 75 to 86 wt% of the silicon dioxide, 2 to 4 wt% of the aluminum oxide, 9 to 16 wt% of the boron trioxide and 3 to 5 wt% of the sodium oxide.

3. The radiation shield filter of claim 1, further comprising at least one additive selected from the group consisting of calcium oxide (CaO), chromium oxide (Cr₂O₃), iron oxide (Fe₂O₃), potassium oxide (K₂O), magnesium oxide (MgO) and zirconium oxide (ZrO₂).

4. A filter assembly for a radiographic apparatus, comprising:
the radiation shield filter of any one of claims 1 to 3; and
a radiation alignment filter, composed of same components as the radiation shield filter, laid over a front of the radiation shield filter, and having fine holes formed in a surface thereof.

5. The filter assembly of claim 4, wherein the fine holes formed in the radiation alignment filter have a size of 40 to 50 *µ*m and an interval of 120 to 170 *µ*m therebetween.

6. The filter assembly of claim 4, wherein a total thickness of the radiation shield filter and the radiation alignment filter is 10 mm or less.

7. The filter assembly of claim 6, wherein the radiation shield filter has a thickness of 4 to 6 mm and the radiation alignment filter has a thickness of 4 to 6 mm.
